# EUROPEAN PATENT SPECIFICATION

(11) **EP 1 304 957 B1**
(45) Date of publication and mention of the grant of the patent: **21.06.2006**
(21) Application number: 01965758.4
(22) Date of filing: 25.07.2001
(51) Int. Cl.: A61B 5/0215

(54) **CATHETER FOR MEASURING PRESSURE**
KATHETER ZUR DRUCKMESSUNG
CATHETER DE MESURE DE LA TENSION

(30) Priority: 26.07.2000 NL 1015814
(43) Date of publication of application: 02.05.2003
(73) Proprietor: Fornix Medical Systems Holding B.V., 6641 SZ Beuningen (NL)
(72) Inventor: DIJKMAN, Gerrat, NL-7524 RJ Enschede (NL)
(74) Representative: Prins, Adrianus Willem
(86) International application number: PCT/NL2001/000575
(87) International publication number: WO 2002/007595

(56) References cited:
- EP-A- 0 864 293
- WO-A-98/32373
- DE-A- 3 207 044
- DE-A- 3 603 463
- US-A- 4 517 844
- US-A- 4 608 996
- US-A- 4 819 653
- US-A- 4 960 127

## Description

The invention relates to a catheter for performing an in vivo pressure measurement, comprising a catheter tube with at least one channel for a pressure-conducting liquid, a pressure sensor for placing in contact with the liquid, and a liquid space provided with at least two ports, said liquid space being a pressure chamber, wherein the pressure sensor is accommodated, wherein the at least one channel is connected to a first port.

A catheter is known wherein the pressure sensor is arranged in a measuring arrangement and is in contact via a pressure-conducting membrane with liquid in a liquid conduit connected to the third port of a three-way tap provided with four ports, wherein the first port can optionally also be connected to the fourth port which is connected to a flushing line.

During a pressure measurement with the known catheter the catheter tube is flushed and filled with water, wherein the first and fourth port are interconnected. The catheter tube is then introduced into the patient at the intended position, for instance the urinary bladder, and water is introduced into the bladder via a channel ("lumen") present for this purpose in the catheter tube. The signal of the pressure sensor is subsequently set to zero using a compensation signal in a situation where the pressure on the sensor equals the ambient pressure. The third and the second port are interconnected for this purpose. After this so-called balancing of the sensor signal, the first and the third port are interconnected and the pressure in the patient, in this case in his/her bladder, can be measured.

The known catheter has the advantage that it has a low purchase price and can be supplied in sterile state.

The use of the known catheter does have drawbacks. The insertion and venting of the catheter takes a relatively long time due to the inherent use of many components, such as a pressure tube, a tap operating as three-way tap and having four ports and, in the case a disposable pressure tube is used, a pressure-conducting medium, for instance a so-called "dome", in order to separate the pressure sensor to be reused from the part of the catheter to be discarded after use. Another significant drawback is inherently related to the applied measurement technique; because the pressure is measured at a fixed position relatively far outside the patient at the same height as the patient, the measurement result greatly depends upon the degree to which the patient can remain in the same position between balancing of the pressure sensor and the actual measurement.

It is an object of the invention to provide a catheter which, while retaining the advantages of the known catheter, does not have its drawbacks.

This objective is achieved, and other advantages gained, with a catheter of the above described type, wherein according to the invention the pressure sensor is accommodated in the other of the at least two ports.

A device of the type as described in the preamble is known from US 4,608,996 The known fluid control device comprises a housing having three ports, one of which is connectable to a catheter tube for supplying blood from a patient to the housing. The housing further comprises an integrally formed pressure transducer chamber extending out of the housing. In the pressure transducer chamber a pressure sensor is accommodated that is connected to the ports by a set of capillary tubes.

In a catheter according to the invention the pressure sensor is accommodated in a pressure chamber which is used to balance the sensor, which tap is placed directly on the end of the catheter tube and is thus situated at the same height as the catheter tube. Consequently the measurement result does not depend on the extent to which a patient can remain in the same position, since a movement of the patient cannot result in a difference in height of sensor and catheter tube.

Additional advantages of a catheter according to the invention are the reduced number of components, resulting in a reduced environmental impact, and the extremely short time required to insert the catheter.

In a preferred embodiment the pressure chamber is formed, essentially by the two ports being in close proximity to one another.

In a further preferred embodiment the two ports are directly connected to one another.

In a alternative preferred embodiment the two ports are connected to one the two ports are connected to one another through a tap provided with a further port, wherein the at least one channel is connected to a the first port, and a the second port is in open communication with the environment, and the tap is adapted for connection of the third port as required to the first or the second port, and wherein the pressure sensor is provided in the third port in close proximity to the first port. By means of this embodiment the catheter can be balanced in a very short time, even after being introduced into the patient.

In a second embodiment of a catheter according to the invention, wherein the catheter tube comprises at least two channels, wherein each channel is connected to the first port of a pressure chamber provided with at least two ports and wherein a pressure sensor is provided in each pressure chamber.

Using such a catheter it is possible to perform pressure measurements at a number of locations simultaneously in the relevant organ of a patient.

Depending on the embodiment and the number of lumina, a catheter according to the invention is for instance suitable for measuring pressure in bladder, urethra or rectum (for which purpose the catheter tube is provided with a balloon for filling with water). The catheter is likewise suitable for invasive measurement of blood pressure or for measuring cranial pressure.

The invention also relates to a pressure chamber as described as component of a catheter according to the invention.

The invention likewise relates to a set of a catheter according to the invention and a connector unit comprising a housing provided with one or more recesses which are each intended to receive a pressure chamber of the a catheter wherein electronic means for connecting to the pressure sensor are arranged in the housing. The invention is as defined in the appended set of claims.

The invention will now be elucidated hereinbelow on the basis of embodiments and with reference to the drawings.

In the drawings
Fig. 1 shows a perspective bottom view of a two-way tap provided with a pressure sensor for a catheter according to the invention,
Fig. 2 shows in cut-away perspective view the two-way tap depicted in fig. 1,
Fig. 3a, 3b show schematic views of the positions of the tap depicted in fig. 1,
Fig. 4 shows a first embodiment of a catheter tube connectable to a two-way tap according to the invention,
Fig. 5 shows a second embodiment of a catheter tube connectable to a two-way tap according to the invention,
Fig. 6 shows a third embodiment of a catheter tube connectable to a two-way tap according to the invention,
Fig. 7 shows a fourth embodiment of a catheter tube connectable to a two-way tap according to the invention,
Fig. 8 shows a fifth embodiment of a catheter tube as component of a catheter according to the invention, and
Fig. 9A shows in schematic perspective view a connector unit for reading the pressure sensor(s), and
Fig. 9B shows a schematic cross-section of the connector unit of figure 9A,
Fig. 10 shows a pressure chamber provided with an integrated pressure sensor for a catheter according to the invention.

Corresponding components are designated in the figures with the same reference numerals.

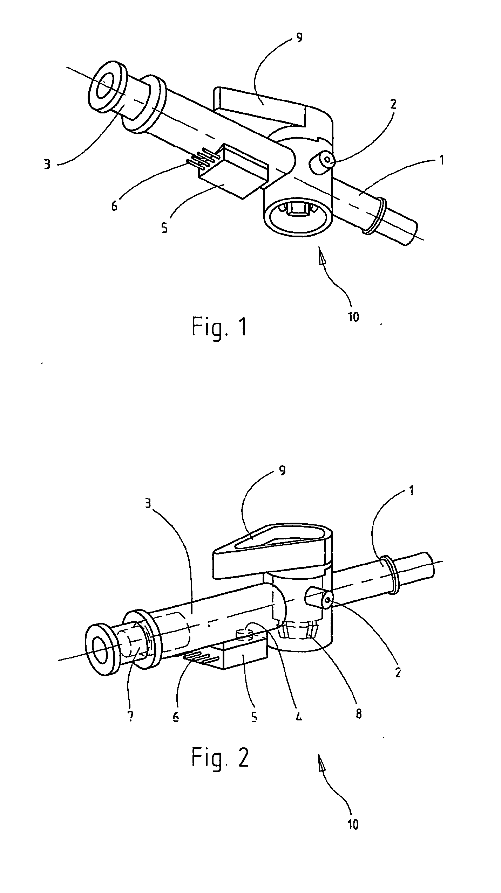

Fig. 1 and 2 show a two-way tap 10 provided with three ports 1, 2, 3, wherein a lumen of a catheter tube (not shown) can be connected to the first port 1, and the second port 2 is in open communication with the environment. Situated in the third port 3 in close proximity to the first port is the pressure-sensitive surface 4 of a pressure sensor 5 which is provided with connecting pins 6. The third port 3 is closed by means of a non-return valve 7. The figure further shows the plug 8, which is provided with bores in T-shape, and the arrow-shaped handle 9 of tap 10.

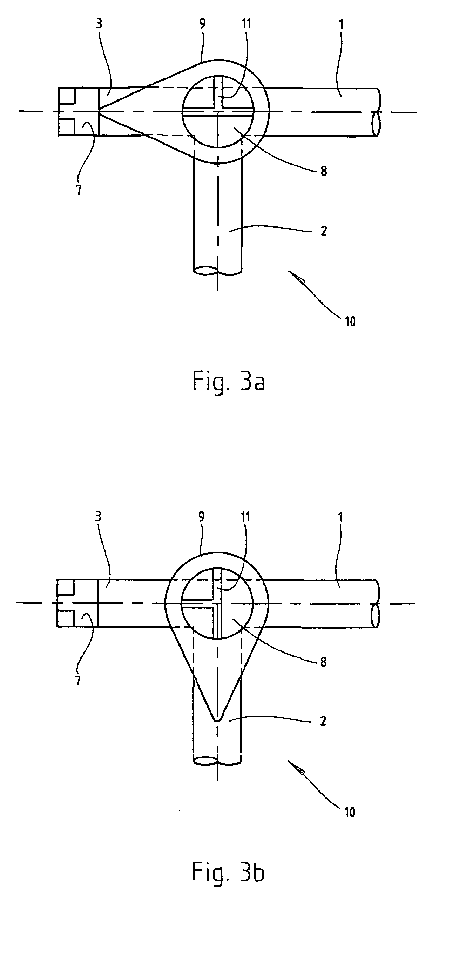

Fig. 3a shows the two-way tap 10 in schematic top view at the start of a pressure measurement, wherein the catheter tube (not shown) connected to the first port 1 and not yet introduced into the patient is flushed with liquid injected into the first channel using a syringe via non-return valve 7 and T-shaped channel 11 in plug 8. After flushing, the handle 9 is rotated a quarter-turn to the left to the position shown in fig. 3b.

The use of a non-return valve eliminates the need for an additional tap for the purpose of flushing and venting the catheter.

Fig. 3b shows two-way tap 10 in the situation prior to balancing of the pressure sensor (not shown) arranged in the third port 3, wherein third port 3 is in open communication with the environment via T-shaped channel 11 and second channel 2. After balancing, the handle 9 is rotated a quarter-turn to the right back into the situation shown in fig. 3a, wherein third port 3 and the pressure sensor placed therein is in open communication with the first port 1 and the catheter lumen connected thereto.

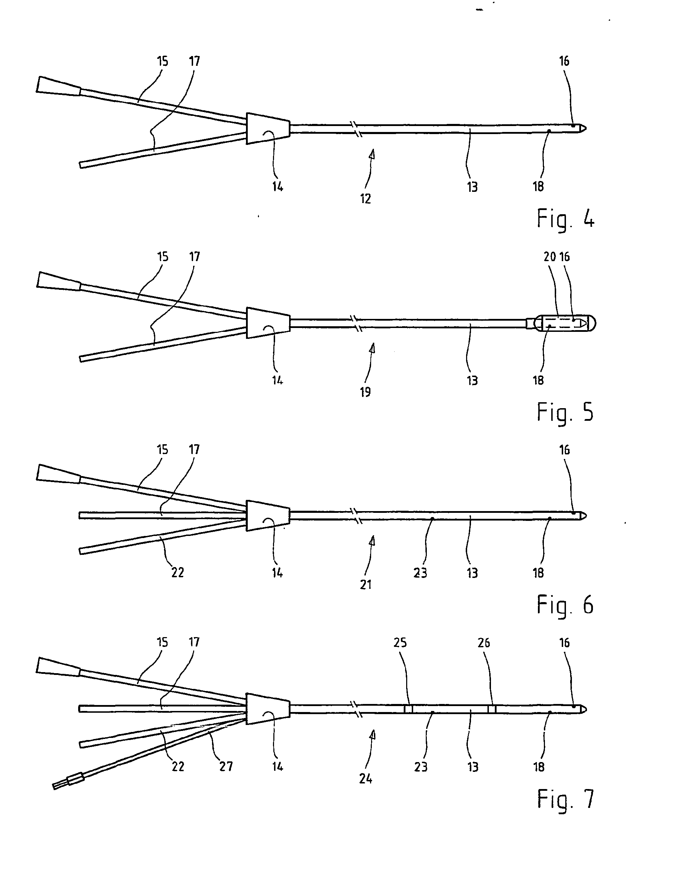

Fig. 4 shows a catheter tube 12 for measuring pressure in a part of a patient which is naturally filled with liquid, for instance a bladder or urethra, with a part 13 for introducing into a patient which is provided with a manifold 14, a first lumen 15 which debouches at the end of part 13 into an opening 16 for introducing liquid into the patient, and a measuring lumen 17 which can be coupled to the third port 3 of two-way tap 10 shown in figures 1-3b. Via an opening 18 in tube part 13 the measuring lumen 17 is in open communication with the liquid introduced into the patient via opening 16.

Fig. 5 shows a catheter tube 19 for measuring the pressure in a part of a patient which naturally contains no water, for instance the rectum. The end of tube part 13 with openings 16, 18 is enclosed by a balloon of polyurethane with a diameter of about 1 cm, which is filled with liquid via the first lumen 15 and opening 16, the pressure of which is measured via opening 18 and the liquid in lumen 17.

Fig. 6 shows a catheter tube 21 provided with a second measuring lumen 22 which via a second measuring opening 23 in tube part 13 is in open communication at a second position with the liquid introduced into the patient via opening 16. The second measuring lumen 22 can be coupled to the third port 3 of a second two-way tap 10.

Fig. 7 shows a catheter tube 24 which corresponds with the catheter tube 21 of fig. 6 but which is however augmented with two electrodes 25, 26 round tube part 13, provided with a connecting cable 27, to measure the electrical resistance of the section between electrodes 25, 26 so as to enable detection of possible leakage of liquid, for instance urine into the urethra.

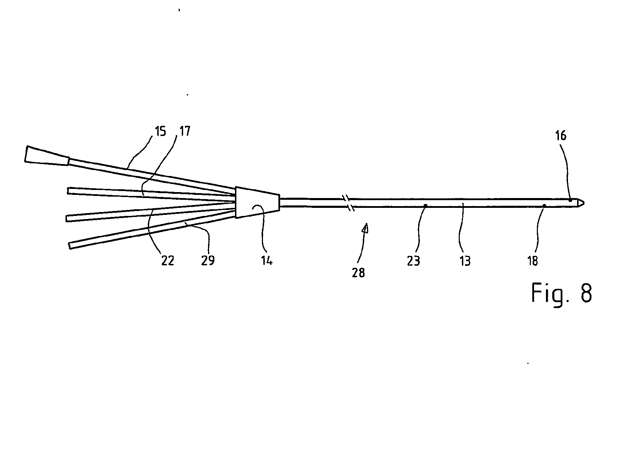

Fig. 8 shows a catheter tube 28 which compared to the catheter tube shown in fig. 6 is augmented with a third measuring lumen 29 which via a third measuring opening (not shown) in tube part 13 is in open communication at a third position with the liquid introduced into the patient via opening 16. The third measuring lumen 29 can be coupled to the third port 3 of a third two-way tap 10. The number of measuring lumina with pressure sensors for reading can of course be further increased or reduced as desired.

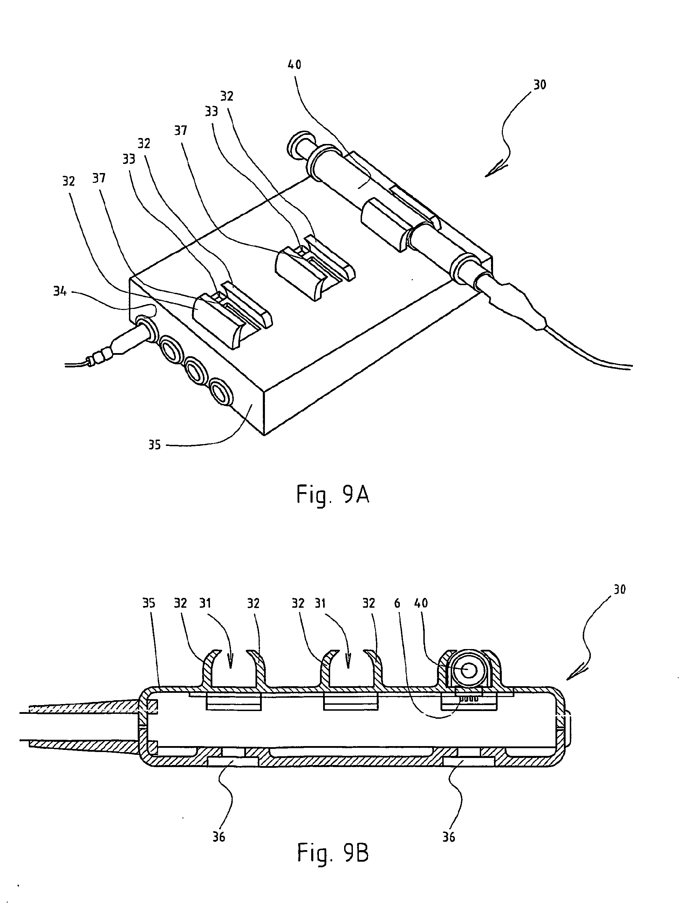

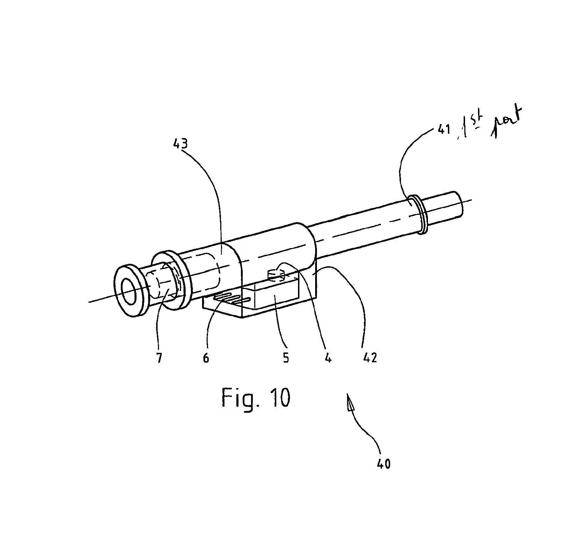

Fig. 9A shows a connector unit 30 with a housing 35 which is provided with a number of recesses 31, each of which is intended to receive a port 3, 43 of a pressure chamber 10, 40 of a catheter according to the invention (see figures 1, 2 and 10). An opening 33 for pressure sensor 4,5 is arranged in each recess. Recesses 31 are further provided with standing edges 32 for fixing the ports of the pressure chambers in the correct position. The deeper levels 37 are arranged for reception of the pressure sensors, possibly provided with a protecting housing, such as housing 42 in figure 10.

Fig. 9B shows connector unit 30 in cross-section. Here can be seen that pressure sensor 4,5 is provided with pins 6 which protrude inward into the housing to enable placing thereof in contact with further electronic means (not shown) for reading the measurement values of the pressure sensor. Connector unit 30 is provided on the side with electrical connections 34. Diverse suitable electronic means for reading such commercially available pressure sensors are likewise commercially obtainable and are therefore known to a skilled person in this technical field. The connector unit can for instance comprise connecting cables for connection of the pressure sensors to a separate data-processing unit. Alternatively, a pre-amplifier can be arranged in the connector unit for a first processing of the data from the pressure sensors. The processed data is then transmitted via connecting cables to a separate data-processing unit.

Connector unit 30 can be attached to a patient by means of a number of surface electrodes and is suitable for both stationary and ambulatory use. For this purpose openings 36 are arranged in housing 35 for receiving the usual fastening means present on known surface electrodes. An earth connection is preferably arranged on the fastening means. In the shown preferred embodiment the connector unit 30 is suitable for simultaneous reading of three pressure sensors. The three pressure sensors for reading can provide measurement values of for instance the bladder pressure (twice) and the urethra pressure, which can be measured simultaneously using a catheter according to the invention. Fig. 8 shows a preferred embodiment of a catheter according to the invention which is suitable for this purpose.

Fig. 10 shows a pressure chamber 40 which is provided with two ports 41 and 43. Port 41 is intended for connection to a lumen of a catheter tube. Port 43 is provided with a non-return valve 7. A pressure sensor 4,5 is received in pressure chamber 40. Pressure chamber 40 is partially provided with a housing 42 to protect the pins 6 of pressure sensor 4,5. If desired, such a protecting housing can also be applied to pressure chamber 10 in figures 1 and 2.

Pressure chamber 40 is a simpler embodiment of the invention. The foregoing figures show the preferred embodiment with two-way tap. This provides the additional option of connecting the first port to the environment so as to still enable balancing of the catheter even after it has been introduced into the patient. This is an option and not essential as such to the invention. It is therefore noted with emphasis that everywhere in the preceding description "(two-way) tap" can be understood to mean "pressure chamber".

In summary, the invention teaches the connection of a pressure chamber to the lumen of a catheter tube and the integration of a pressure sensor in the pressure chamber. The pressure chamber can optionally be provided with a two-way tap. The pressure chamber and the lumen are preferably connected fixedly to each other, for instance by means of glueing.

In a catheter according to the invention the number of lumina with pressure chamber and sensor integrated therein can be chosen as desired, so that multiple measurements can be performed simultaneously. The catheter according to the invention is a disposable article and is highly suitable for medical applications in general and urological applications in particular.

It is noted that the shown embodiments are in no way intended to limit the scope of the invention, but serve only for purposes of elucidation. The invention is not therefore limited to the shown and described embodiments, but extends generally to any embodiment which falls within the scope of the appended claims as seen in the light of the foregoing description and drawings. This particularly includes all combinations of the shown and discussed embodiments which can be realized by a skilled person. While the invention is outlined in the context of urological applications, it is also particularly suitable for many other medical applications, such as in the field of gastro-enterology or for measuring cranial pressure, for instance after an invasive treatment.

## Claims

1. Catheter for performing an in vivo pressure measurement, comprising a catheter tube (12, 19, 21, 24, 28) with at least one channel (17) for a pressure-conducting medium, a pressure sensor (4, 5) for placing in contact with the medium, and a liquid space (10, 40) provided with at least two ports (1, 3, 41, 43), wherein the at least one channel (17) is connected to a first port (1), wherein the liquid space is a pressure chamber (1) in which the pressure sensor (4, 5) is accommodated, **characterized in that** a second port is provided for introducing a liquid into said liquid space, said sensor being situated between said first and second port (1, 3, 41, 43).

2. Catheter according to claim 1, wherein a non-return valve is provided in at least one of said ports.

3. Catheter according to claim 2, wherein said non-return valve (7) is provided in said second port.

4. Catheter according to any one of the preceding claims, wherein said catheter tube has at least one further channel or lumen (15, 22, 27, 29), at least one of said further channels or lumen (15, 22, 27, 29) being provided for introducing a liquid into said catheter tube distanced from said liquid space.

5. Catheter according to any one of the preceding claims, wherein said catheter tube comprises a part (13) for introducing into a patient, having an end comprising a first opening (16) and a second opening (18), wherein said first opening is connected to said at least one channel (17) connected to said first port (1) and said second opening is connected to a second channel (15, 22, 27, 29).

6. Catheter according to claim 4 or 5, wherein said catheter tube comprises a manifold (14).

7. Catheter according to any one of the preceding claims, wherein the pressure chamber is provided with a tap (10) provided with three ports (1, 2, 3), wherein the at least one channel (17) is connected to a first port (1) and a second port (2) is in open communication with the environment, and the tap (10) is adapted for connection of the third port (3) as required to the first (1) or the second port (2), and wherein the pressure sensor (4, 5) is provided in the third port (3) in close proximity to the first port (1).

8. Catheter according to any one of the preceding claims, wherein the catheter tube (19, 21, 23) comprises at least two channels (17, 22), wherein each channel (17, 22) is connected to the first port (1) of a pressure chamber (10, 40) provided with at least two ports, and wherein a pressure sensor (4, 5) is provided in each pressure chamber (10, 40).

9. Catheter according to any one of the preceding claims, wherein said pressure chamber is shaped as a substantially straight tubular housing having two ports (1, 3; 41, 43) at opposite ends of said housing, wherein said pressure sensor (4, 5) is provided in a side wall of said housing.

10. Catheter according to claim 9, wherein said housing comprises a tap (10) between said opposite ports (1, 3; 41, 43), said sensor (4, 5) being provided between said tap (10) and the said port opposite said first port (1; 41).

11. Catheter according to claim 9 or 10, wherein said port (3; 43) opposite said first port (1, 41) is provided with a no-return valve (7).

12. Pressure chamber for connecting to a channel (17) of a catheter tube (12, 19, 21, 24, 28), comprising a housing having a first port (1; 41) and a third port (3; 43) at opposite sides of said housing, said first port (1; 41) being arranged for connecting to said channel (17) and said third port (3; 43) being arranged for introducing liquid into said pressure chamber, **characterized in** a pressure sensor being provided in said pressure chamber, between said first port (1; 41) and said third port (3; 43), wherein a tap (10) is provided between said first (1; 41) and third port (3; 43), said tap being adapted for connection of the third port (3) as required to the first port (1) or a second port (2) in open communication with the environment, said pressure sensor being provided between said second port (2) and said third port (3).

13. Set of a catheter according to any one of claims 1-11 and a connector unit comprising a housing (30) provided with one or more recesses (31) which are each intended to receive a pressure chamber (10, 40) of a catheter as claimed in any of the foregoing claims, wherein electronic means for connecting to the pressure sensor (4, 5) are arranged in the housing.

## Patentansprüche

1. Katheter zur Durchführung einer in-vivo-Druckmessung, mit einem Katheterröhrchen (12,19,21,24,28) mit mindestens einem Kanal (17) für ein druckführendes Medium, einem Drucksensor (4,5), der mit dem Medium in Kontakt gebracht wird, und einem Flüssigkeitsraum (10,40) mit mindestens zwei Ports (1,3,41,43), wobei der mindestens eine Kanal (17) mit einem ersten Port (1) verbunden ist, wobei der Flüssigkeitsraum eine Druckkammer (1) ist, in dem der Drucksensor (4,5) angeordnet ist,
**dadurch gekennzeichnet, dass**
ein Port zum Einleiten einer Flüssigkeit in den Flüssigkeitsraum vorgesehen ist, wobei der zweite Sensor zwischen dem ersten und dem zweiten Port (1,3,41,43) angeordnet ist.

2. Katheter nach Anspruch 1, bei dem ein Rückschlagventil in mindestens einem der Ports angeordnet ist.

3. Katheter nach Anspruch 2, bei dem das Rückschlagventil (7) in dem zweiten Port angeordnet ist.

4. Katheter nach einem der vorhergehenden Ansprüche, bei dem das Katheterröhrchen mindestens einen weiteren Kanal oder ein weiteres Lumen (15,22,27,29) aufweist, wobei mindestens einer/eines der weiteren Kanäle oder Lumen (15,22,27,29) zum Einleiten einer Flüssigkeit in das von dem Flüssigkeitsraum beabstandete Katheterröhrchen vorgesehen ist.

5. Katheter nach einem der vorhergehenden Ansprüche, bei dem das Katheterröhrchen mit einem Teil (13) zum Einführen in den Körper eines Patienten versehen ist, wobei der Teil (13) ein Ende mit einer ersten Öffnung (16) und einer zweiten Öffnung (18) aufweist, wobei die erste Öffnung mit dem mindestens einen Kanal (17) verbunden ist, welcher mit dem ersten Port (1) verbunden ist, und die zweite Öffnung mit einem zweiten Kanal (15,22,27,29) verbunden ist.

6. Katheter nach Anspruch 4 oder 5, bei dem das Katheterröhrchen einen Verteiler (14) aufweist.

7. Katheter nach einem der vorhergehenden Ansprüche, bei dem die Druckkammer mit einer Entnahmeeinrichtung (10) mit drei Ports (1,2,3) versehen ist, wobei der mindestens eine Kanal (17) mit einem ersten Port (1) verbunden ist und ein zweiter Port (2) in offener Verbindung mit der Umgebung steht, und die Entnahmeeinrichtung (10) zum Verbinden des dritten Ports (3) je nach Bedarf mit dem ersten (1) oder dem zweiten Port (2) angepasst ist, und wobei der Drucksensor (4,5) in dem dritten Port (3) sehr nahe dem ersten Port (1) angeordnet ist.

8. Katheter nach einem der vorhergehenden Ansprüche, bei dem das Katheterröhrchen (19,21,28) mindestens zwei Kanäle (17,22) aufweist, wobei jeder Kanal (17,22) mit dem ersten Port (1) einer mindestens zwei Ports aufweisenden Druckkammer (10,40) verbunden ist, und wobei ein Drucksensor (4,5) in jeder Druckkammer (10,40) angeordnet ist.

9. Katheter nach einem der vorhergehenden Ansprüche, bei dem die Druckkammer als im Wesentlichen gerades röhrförmiges Gehäuse mit zwei Ports (1,3;41,43) an entgegengesetzten Enden des Gehäuses ausgebildet ist, wobei der Drucksensor (4,5) in einer Seitenwand des Gehäuses angeordnet ist.

10. Katheter nach Anspruch 9, bei dem das Gehäuse eine Entnahmeeinrichtung (10) zwischen den einander gegenüberliegenden Ports (1,3;41,43) aufweist, wobei der Sensor (4,5) zwischen der Entnahmeeinrichtung (10) und dem dem ersten Port (1;41) gegenüberliegenden Port angeordnet ist.

11. Katheter nach Anspruch 9 oder 10, bei dem der dem ersten Port (1;41) gegenüberliegende Port (3;43) mit einem Rückschlagventil (7) versehen ist.

12. Druckkammer zum Verbinden mit einem Kanal (17) eines Katheterröhrchens (12,19,21,24,28), wobei die Druckkammer ein Gehäuse mit einem ersten Port (1;41) und einem dritten Port (3;43) auf entgegengesetzten Seiten des Gehäuses aufweist, wobei der erste Port (1;41) zum Verbinden mit dem Kanal (17) vorgesehen ist und der dritte Port (3;43) zum Einleiten von Flüssigkeit in die Druckkammer vorgesehen ist,
**dadurch gekennzeichnet, dass**
ein Drucksensor in der Druckkammer zwischen dem ersten Port (1;41) und dem dritten Port (3;43) angeordnet ist, wobei eine Entnahmeeinrichtung (10) zwischen dem ersten (1;41) und dem dritten Port (3;43) angeordnet ist, wobei die Entnahmeeinrichtung zum Verbinden des dritten Ports (3) je nach Bedarf mit dem ersten Port (1) oder einem zweiten Port (2), der in offener Verbindung mit der Umgebung steht, angepasst ist, wobei der Drucksensor zwischen dem zweiten Port (2) und dem dritten Port (3) angeordnet ist.

13. Katheter-Set nach einem der Ansprüche 1-11 und Verbindungseinheit mit einem Gehäuse (30) mit einer oder mehreren Ausnehmungen (31), die zum Aufnehmen einer Druckkammer (10,40) eines Katheters nach einem der vorhergehenden Ansprüche vorgesehen sind, wobei Elektronikeinrichtungen zum Verbinden mit dem Drucksensor (4,5) in dem Gehäuse angeordnet sind.

## Revendications

1. Cathéter pour réaliser une mesure de pression in vivo, comprenant un tube de cathéter (12, 19, 21, 24, 28) avec au moins un canal (17) pour un milieu conduisant la pression, un capteur de pression (4, 5) destiné à être mis en contact avec le milieu, et un espace à liquide (10, 40) muni d'au moins deux orifices (1, 3, 41, 43), ou le au moins un canal (17) est relié à un premier orifice (1), où l'espace à liquide est une chambre à pression (1) dans laquelle le capteur de pression (4,6) est reçu, **caractérisé en ce qu'**un second orifice est prévu pour introduire un liquide dans ledit espace à liquide, ledit capteur étant situé entre lesdits premier et second orifices (1, 3,'41, 43).

2. Cathéter selon la revendication 1 où un clapet anti-retour est prévu dans au moins l'un desdits orifices.

3. Cathéter selon la revendication 2 où ledit clapet anti-retour (7) est prévu dans ledit second orifice.

4. Cathéter selon l'une quelconque des revendications précédentes où ledit tube de cathéter a au moins un autre canal ou lumière (15, 22, 27, 29), au moins l'un desdits autres canaux ou lumières (15,22,27,29) étant prévu pour l'introduction d'un liquide dans ledit tube de cathéter à distance dudit espace à liquide.

5. Cathéter selon l'une quelconque des revendications précédentes où ledit tube de cathéter comprend une partie (13) pour l'introduction dans un patient, ayant une extrémité comprenant une première ouverture (16) et une seconde ouverture (18), où ladite première ouverture est reliée audit au moins un canal (17) relié audit premier orifice (1) et ladite seconde ouverture est reliée à un second canal (15, 22, 27, 29).

6. Cathéter selon la revendication 4 ou 5 où ledit tube de cathéter comprend un collecteur (14).

7. Cathéter selon l'une quelconque des revendications précédentes où la chambre à pression est munie d'un robinet (10) muni de trois orifices (1, 2, 3), où le au moins un canal (17) est relié à un premier orifice (1) et un second orifice (2) est en communication ouverte avec l'environnement, et le robinet (10) est adapté pour la connexion du troisième orifice (3) selon ce qui est nécessaire au premier (1) ou au second orifice (2), et où le capteur de pression (4, 5) est prévu dans le troisième orifice (3) à proximité immédiate du premier orifice (1).

8. Cathéter selon l'une quelconque des revendications précédentes où le tube de cathéter (19, 21, 23) comprend au moins deux canaux (17, 22), où chaque canal (17, 22) est relié au premier orifice (1) d'une chambre à pression (10, 40) munie d'au moins deux orifices, et où un capteur de pression (4, 5) est prévu dans chaque chambre à pression (10, 40).

9. Cathéter selon l'une quelconque des revendications précédentes où ladite chambre à pression est sous forme d'un boîtier tubulaire sensiblement rectiligne ayant deux orifices (1, 3; 41, 43) à des extrémités opposées dudit boîtier ou ledit capteur de pression (4, 5) est disposé dans une parol latérale dudit boîtier.

10. Cathéter selon la revendication 9 où ledit boîtier comprend un robinet (10) entre lesdits orifices opposés (1, 3 ; 41, 43), ledit capteur (4, 5) étant disposé entre ledit robinet (10) et ledit orifice opposé audit premier orifice (1; 41).

11. Cathéter selon la revendication 9 ou 10, où ledit orifice (3; 43) opposé audit premier orifice (1,41) est muni d'un clapet anti-retour (7).

12. Chambre à pression destinée à être reliée à un canal (17) d'un tube de cathéter (12,19,21,24,28), comprenant un boîtier ayant un premier orifice (1 ; 41) et un troisième orifice (3 ; 43) à des côtés opposés dudit boîtier, ledit premier orifice (1 ; 41) étant disposé pour la connexion audit canal (17) et ledit troisième orifice (3 ; 43) étant disposé pour introduire un liquide dans ladite chambre à pression, **caractérisée en ce qu'**un capteur de pression est prévu dans ladite chambre à pression, entre ledit premier orifice (1; 41) et ledit troisième orifice (3 ; 43), où un robinet (10) est prévu entre lesdits premier (1 ; 41) et troisième orifices (3 ;43) ; ledit robinet étant adapté pour la connexion du troisième orifice (3) selon ce qui est nécessaire au premier orifice (1) ou un second orifice (2) en communication ouverte avec l'environnement, ledit capteur de pression étant disposé entre ledit second orifice (2) et ledit troisième orifice (3).

13. Ensemble d'un cathéter selon l'une quelconque des revendications 1-11 et d'une unité de connecteur comprenant un boîtier (30) muni d'un ou plusieurs évidements (31) qui sont destinés chacun à recevoir une chambre à pression (10; 40) d'un cathéter selon l'une quiconque des revendications précédentes, où des moyens électroniques destinés à être reliés au capteur de pression (4, 5) sont disposés dans le boîtier.
